Europäisches Patentamt

European Patent Office

Office européen des brevets

(19)

(11) Veröffentlichungsnummer: **0 154 372**
**A2**

(12) # EUROPÄISCHE PATENTANMELDUNG

(21) Anmeldenummer: 85200215.3

(22) Anmeldetag: 20.02.85

(51) Int. Cl.⁴: **A 61 B 6/02**

(30) Priorität: 24.02.84 DE 3406717

(43) Veröffentlichungstag der Anmeldung:
**11.09.85 Patentblatt 85/37**

(84) Benannte Vertragsstaaten:
**DE FR GB IT NL**

(71) Anmelder: **Philips Patentverwaltung GmbH**
**Billstrasse 80**
**D-2000 Hamburg 28(DE)**

(84) Benannte Vertragsstaaten:
**DE**

(71) Anmelder: **N.V. Philips' Gloeilampenfabrieken**
**Groenewoudseweg 1**
**NL-5621 BA Eindhoven(NL)**

(84) Benannte Vertragsstaaten:
**FR GB IT NL**

(72) Erfinder: **Hepke, Jürgen**
**Dörpsweg 53**
**D-2000 Hamburg 54(DE)**

(74) Vertreter: **Hartmann, Heinrich, Dipl.-Ing. et al,**
**Philips Patentverwaltung GmbH Billstrasse 80 Postfach**
**10 51 49**
**D-2000 Hamburg 28(DE)**

(54) **Röntgenschichtaufnahmegerät.**

(57) Die Erfindung betrifft ein Röntgenschichtaufnahmegerät, bei dam der Strahler unabhängig voneinander Bewegungen in zwei zueinander senkrechten Richtungen ausführen kann. Der Röntgenstrahler ist in einem ersten Träger in Querrichtung verfahrbar, der an einem zweiten sich in Längsrichtung erstreckenden Träger befestigt ist, der in seiner Hauptausdehnungsrichtung verfahrbar in einem am Boden befestigten Stativ gelagert ist.

EP 0 154 372 A2

./...

Fig.1

"Röntgenschichtaufnahmegerät"

Die Erfindung betrifft ein Röntgenschichtaufnahmegerät mit einem Röntgenstrahler, der in einer horizontalen Ebene in Längsrichtung und davon unabhängig in Querrichtung eines Untersuchungstisches verfahrbar ist und der über eine Lenkstange mit einer Bildaufnahmeeinrichtung gekoppelt ist.

Ein solches Schichtaufnahmegerät ist aus der DE-OS 19 36 915 bekannt. Durch die Verfahrbarkeit des Strahlers in Längsrichtung und unabhängig davon in Querrichtung gestattet ein solches Gerät die Ausführung von beliebigen in einer horizontalen Ebene liegenden Verwischungsfiguren. Bei dem bekannten Gerät ist der Strahler an einem teleskopartigen Deckenstativ befestigt, der von einem ersten Wagen getragen wird, der in sich in Querrichtung erstreckenden Schienen verfahrbar ist, die an einem zweiten Wagen befestigt sind, der in sich in Längsrichtung erstreckenden Schienen verfahrbar ist. Diese letztgenannten Schienen sind an der Decke befestigt. Für ein derartiges Gerät ist eine hinreichend stabile Deckenkonstruktion erforderlich und es müssen Maßnahmen getroffen werden, um zu verhindern, daß sich Erschütterungen während einer Schichtaufnahme nachteilig auf die Abbildungseigenschaften auswirken.

Weiterhin ist aus der DE-PS 12 50 594 ein Schichtaufnahmegerät bekannt, bei dem der Röntgenstrahler an einem Träger befestigt ist, der in einem Führungsteil in Querrichtung verfahrbar ist. Das Führungsteil ist in Längsrichtung einer vertikalen Säule verschiebbar, die ihrerseits an

0154372

Schienen am Boden und an der Decke in Längsrichtung verfahrbar ist. Die Verschiebebewegungen in Längs- und in Querrichtung sind mechanisch miteinander gekoppelt, so daß nur bestimmte Verwischungsfiguren möglich sind. Auch hierbei ist die Aufstellung nur mittels einer Deckenkonstruktion möglich, auch wenn deren Tragfähigkeit geringer sein kann als im vorgenannten Fall. Dafür ist aber eine Bodenschiene erforderlich.

Der Erfindung liegt die Aufgabe zugrunde, ein Röntgengerät so auszugestalten, daß weder eine Deckenkonstruktion noch eine Bodenschiene erforderlich sind.

Diese Aufgabe wird erfindungsgemäß dadurch gelöst, daß der Röntgenstrahler in einemersten Träger in Querrichtung verfahrbar ist, daß der erste Träger am Ende eines zweiten Trägers befestigt ist, der sich in Längsrichtung erstreckt und in dieser Richtung verfahrbar in einem am Boden befestigbaren Stativ gelagert ist.

Die Erfindung wird nachstehend anhand der Zeichnung näher erläutert. Es zeigen

Fig. 1 ein erstes Ausführungsbeispiel
und
Fig. 2 einen Teil eines zweiten Ausführungsbeispiels.

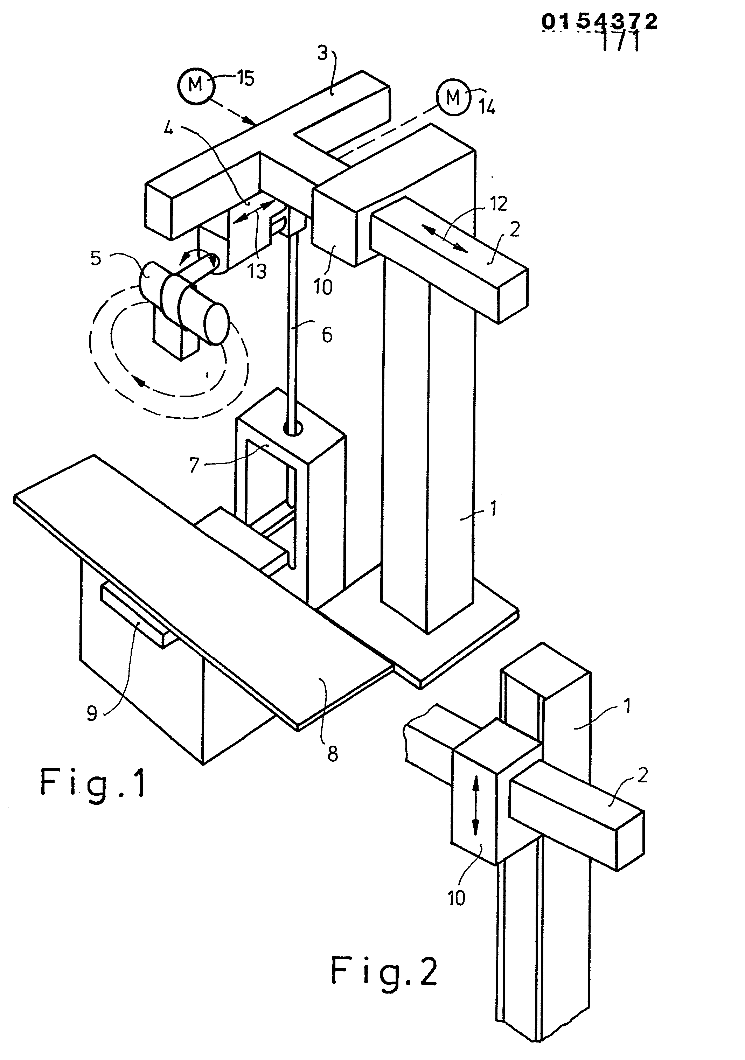

In Fig. 1 ist mit 1 ein feststehendes Stativ in Form einer am Boden befestigten vertikalen Säule bezeichnet, die an ihrem oberen Ende ein abgewinkeltes Führungsteil 11 trägt, in dem ein Träger 2 horizontal und in Richtung des Pfeiles 12, d.h. in Tischlängsrichtung, verschiebbar gelagert ist. An dem einen Ende des sich in Tischlängsrichtung erstreckenden Trägers 2 ist ein sich senkrecht dazu erstreckender Träger 3 befestigt derart, daß die beiden Träger 2 und 3 ein in einer horizontalen Ebene befindliches T bilden. In dem Träger 3 ist ein in dessen Längsrichtung, d.h. in Richtung des Pfeiles 13,verfahrbarer
Wagen 4 geführt, der einen Röntgenstrahler 5 trägt und der
mit dem einen Ende einer Koppelstange 6 gekoppelt ist, die
in einem nicht näher dargestellten Lager in dem Gestell 7
kadarnisch gelagert ist und deren anderes Ende mit einer
eine Filmkassette enthaltenden Filmlade 9 verbunden ist.
Die Filmlade befindet sich unterhalb der Tischplatte 8 des
Untersuchungstisches, auf dem der Patient während der
Untersuchung liegt. Die Längsrichtung der Tischplatte
stimmt mit dem Pfeil 12 überein und die Querrichtung mit
dem Pfeil 13.

Die Verschiebung des Wagens 4 innerhalb des Trägers 3 in
Querrichtung und die Verschiebung des Trägers 2 innerhalb
des Führungsteils 11 in Längsrichtung erfolgt mit Hilfe
der schematisch angedeuteten Motoren 14 und 15. Wenn deren
Drehzahlverläufe als Funktion der Zeit in geeigneter Weise
aufeinander abgestimmt werden, kann der Röntgenstrahler 5
bzw. die mit ihm über die Lenkstange 6 mechanisch gekoppelte Filmlade 9 jede beliebige Verwischungsfigur ausführen. Der Motor 14 für die Querverschiebung kann in dem
Wagen 4 angeordnet sein und in nicht näher dargestellter
Weise eine Mutter antreiben, die auf einer Schraubspindel
läuft, so daß der Wagen bei einer Drehung der Spindel in
Richtung des Pfeiles 13 verschoben wird. Diese Antriebsart
ist u.a. aus der DE-AS 19 36 915 bekannt. Auf ähnliche
Weise wird der Träger 2 mittels des im Führungsteil 11 angeordneten Motors 15 verschoben, der auf eine Mutter einwirkt, die mit einer am Träger 2 befestigten Spindel
zusammenwirkt.

Anders als bei dem bekannten Gerät nach der
DE-AS 19 36 915 besteht zwischen dem Röntgenstrahler und
der Filmlade eine direkte Kopplung, was der Bildqualität

**0154372**

zugute kommt. Eine Deckenkonstruktion ist nicht erforderlich und trotz geringerer Bauform ergibt sich zumindest
die gleiche Stabilität. Das Gerät ist gut von allen Seiten
zugänglich, weil Bodenschienen und dergleichen fehlen.

Die Ausführungsform nach Fig. 2 unterscheidet sich von
derjenigen nach Fig. 1 dadurch, daß der Führungsteil 10,
in dem der Träger 2 gelagert ist, mit der Säule 1 nicht
fest verbunden ist, sondern an dieser vertikal verschiebbar angeordnet ist – ähnlich wie bei dem Schichtaufnahmegerät nach der DE-PS 12 50 594. Dadurch ist es möglich,
für eine Röntgenschichtaufnahme die Höhe der horizontalen
Ebene, in der der Röntgenstrahler 5 bewegt wird, den
jeweiligen Anforderungen anzupassen. Die Einstellbarkeit
des Strahlers in der Höhe ist auch für andere als
Schichtaufnahmen z.B. Aufnahmen auf Wandstativ oder
Aufnahmen mit größerem FFA auf den Tisch von Interesse.

**0154372**

## PATENTANSPRÜCHE

1.    Röntgenschichtaufnahmegerät mit einem Röntgenstrahler, der in einer horizontalen Ebene in Längsrichtung und davon unabhängig in Querrichtung eines Untersuchungstisches verfahrbar ist und der über eine Lenkstange mit einer Bildaufnahmeeinrichtung gekoppelt ist, dadurch gekennzeichnet, daß der Röntgenstrahler (5) in einem ersten Träger (8) in Querrichtung verfahrbar ist, daß der erste Träger am Ende eines zweiten Trägers (2) befestigt ist, der sich in Längsrichtung erstreckt und in dieser Richtung verfahrbar in einem am Boden befestigbaren Stativ (1) gelagert ist.

2.    Röntgenschichtaufnahmegerät nach Anspruch 1, dadurch gekennzeichnet, daß die Verbindung der beiden Träger (2, 3) annähernd eine T-Form hat.

3.    Röntgenschichtaufnahmegerät nach Anspruch 1 oder 2, dadurch gekennzeichnet, daß das Stativ als Vertikalsäule (1) ausgebildet ist, an der ein in vertikaler Richtung verfahrbares Führungsteil (10) angeordnet ist, in dem der zweite Träger (2) gelagert ist.

Fig.1

Fig.2

0154372
171

PHD 84-031